# EUROPÄISCHE PATENTSCHRIFT

(11) **EP 0 666 276 B1**
(45) Veröffentlichungstag und Bekanntmachung des Hinweises auf die Patenterteilung: **06.05.1999**
(21) Anmeldenummer: 95100850.7
(22) Anmeldetag: 23.01.1995
(51) Int. Cl.: C08G 18/78, C08G 18/76

(54) **Oberhalb 5 Grad C flüssige Polyisocyanatmischungen**
Polyisocyanate mixtures which are liquid above 5 C
Mélanges de polyisocyanates liquides au dessus de 5 C

(30) Priorität: 03.02.1994 DE 4403233
(43) Veröffentlichungstag der Anmeldung: 09.08.1995
(73) Patentinhaber: BAYER AG, 51368 Leverkusen (DE)
(72) Erfinder: Schmalstieg, Lutz, Dr., D-50676 Köln (DE); Pedain, Josef, Dr., D-51061 Köln (DE); Mertes, Harald, Dr., D-50670 Köln (DE)

(56) Entgegenhaltungen:
- EP-A- 0 019 375
- GB-A- 2 066 813
- US-A- 4 738 991

## Beschreibung

Die Erfindung betrifft flüssige, Allophanatgruppen enthaltende Polyisocyanatmischungen aus 4,4'-Diphenylmethandiisocyanat, ein Verfahren zu ihrer Herstellung und die Verwendung zur Herstellung von Zweikomponenten-Polyurethan-Beschichtungsmitteln.

Zweikomponenten-Polyurethanbeschichtungsmittel auf Basis Diphenylmethandiisocyanat sind seit langem bekannt und werden z.B. beschrieben in "Wagner Sarx, Lackkunstharze, 5. Auflage, Carl Hanser Verlag, München, 1971, S. 169 und 170.

Zur Herstellung lösungsmittelfreier Zweikomponenten-Polyurethan-Systeme, die es gestatten dicke Beschichtungen in einem Arbeitsgang herzustellen, werden in der Regel flüssige Polyisocyanatgemische der Diphenylmethanreihe als Polyisocyanat-komponente verwendet. Es handelt sich hierbei in der Regel um undestillierte und daher stark gefärbte Polyisocyanatgemische, wie sie in bekannter Weise durch Phosgenierung von Anilin/Formaldehyd-Kondensaten zugänglich sind.

Für dekorative Anwendungen wie z.B. die Fußbodenbeschichtung ist es aber wünschenswert, hellfarbige Diphenylmethandiisocyanatmischungen einzusetzen. Solche flüssigen, hellfarbigen Polyisocyanate sind beispielsweise Mischungen von 4,4'-Diphenylmethandiisocyanat mit größeren Mengen 2,4'-Diphenylmethandiisocyanat. Derartige Mischungen zeigen aber eine schlechte Kristallisationsbeständigkeit bei tiefen Temperaturen, außerdem verschlechtert der 2,4'-Diphenylmethandiisocyanatanteil die Eigenschaften der daraus hergestellten Beschichtungsmaterialien deutlich. Gesucht werden daher hellfarbige, kristallisationsstabile Polyisocyanatgemische, die ausschließlich auf 4,4'-Diphenylmethandiisocyanat basieren und für den genannten Zweck geeignet sind.

Die Herstellung von modifizierten, bei Raumtemperatur flüssigen Polyisocyanatgemischen auf Basis von 4,4'-Diphenylmethandiisocyanat ist bereits bekannt: Zur Herstellung derartiger Gemische werden beispielsweise gemaß DE-PS 1 618 380 1 Mol 4,4'-Diphenylmethandiisocyanat mit 0,1 bis 0,3 Mol Tri-1,2-oxypropylenglykol und/oder Poly-1,2-oxypropylenglykol mit einem Molekulargewicht bis zu 700 zur Reaktion gebracht. Gemaß GB-PS 1 369 334 wird die Modifizierung in zwei Reaktionsstufen durchgeführt und als Modifizierungsmittel Dipropylenglykol oder Polyoxypropylenglykol mit einem Molekulargewicht unter 2000 verwendet. Die DE-OS 2 913 126 beschreibt Diphenylmethandiisocyaantzusammensetzungen, in denen 10 bis 35 Gew.-% der Isocyanatgruppen mit einem Gemisch aus mindestens 3 Alkylenglykolen umgesetzt werden, wobei eines dieser Glykole Di-, Tri- oder Polypropylenglykol ist. In der DE-OS 2 404 166 werden hingegen als Modifizierungsmittel Gemische aus Polyoxyethylenglykol oder Polyoxypropylenglykol mit einem durchschnittlichen Molekulargewicht kleiner als 650 und mindestens einem Alkylenglykol mit mindestens 3 C-Atomen genannt. Die DE-OS 2 346 996 betrifft schließlich Diphenylmethandiisocyanat-Zusammensetzungen, bei denen 10 bis 35 Gew.-% der Isocyanatgruppen mit einem handelsüblichen Polyethylenglykol umgesetzt wurden. Gemaß EP-A 0 341 515 wird 4,4'-Diphenylmethandiisocyanat mit Gemischen aus Aminopolyethern und Tripropylenglykol modifiziert. Aus der GB-A 2 066 813 sind solche Mischungen aus Herstellung steifer Polyisocyanuratschaüme bekannt.

Alle Polyisocyanatgemische dieser Veröffentlichungen enthalten wegen der Mitverwendung von Polyetherpolyolen als Modifizierungsmittel eingebaute Etherstrukturen. Dies ist jedoch aus anwendungstechnischer Sicht von Nachteil: Zum einen führt der Einbau solcher Polyole zu einer oft unerwünschten Elastifizierung der Endprodukte, zum anderen sind derartige Polyoxyalkylenoxidstrukturen bekanntlich empfindlich gegen oxidativen Abbau.

Flüssige Polyisocyanatgemische auf Basis 4,4'-Diphenylmethandiisocyanat, die keine Etherstrukturen enthalten, lassen sich durch anteilige Carbodiimidisierung von 4,4'-Diphenylmethandiisocyanat erhalten, so wie dies beispielhaft in DE-PS 1 092 007, DE-OS 2 537 685, US-PS 4 014 935, US-PS 4 088 665 oder US-PS 4 154 752 beschrieben ist. Auch diese Methode ist mit Nachteilen behaftet: Produktgemische, die nur einen geringen Anteil an Carbodiimidstrukturen aufweisen, zeigen zwar eine hinreichend niedrige Viskosität, leiden aber unter einer schlechten Kristallisationsbeständigkeit, insbesondere bei Temperaturen unter 10°C. Produktgemische, die einen größeren Anteil an Carbodiimidstrukturen aufweisen, zeigen dagegen befriedigende Kristallisationsbeständigkeit, haben aber eine für viele Anwendungen zu hohe Viskosität (vgl. z.B. DE-OS 2 537 685, Bsp. 1 bis 3).

Um diese Schwierigkeiten zu überwinden wird daher vielfach zusätzlich zur Carbodiimidmodifizierung eine Modifizierung mit Polyalkylenoxidpolyolen vorgenommen, was aber mit den beschriebenen Nachteilen verbunden ist.

Die Aufgabe der vorliegenden Erfindung bestand folglich darin, bei 5°C kristallisationsstabile, niedrigviskose Polyisocyanatmischungen auf Basis 4,4'-Diphenylmethandiisocyanat bereitzustellen, die keine Ether- bzw. Polyalkylenoxidstrukturen enthalten. Diese Aufgabe konnte durch Bereitstellung der nachstehend näher charakterisierten Allophanatgruppen aufweisenden Polyisocyanatmischungen gelöst werden.

Hier und im folgenden steht "4,4'-Diphenylmethandiisocyanat" sowohl für das reine 4,4'-Isomer als auch für dessen Gemische mit bis zu 1,9 Gew.-% an 2,4'-und/oder bis zu 0,5 Gew.-% an 2,2'-Diphenylmethandiisocyanat, wobei sich diese Prozentangaben jeweils auf das Gesamtgemisch beziehen. Vorzugsweise besteht das als Ausgangsmaterial eingesetzte "4,4'-Diphenylmethandiisocyanat" zumindest zu 98,5 % aus dem 4,4'-Isomeren.

Gegenstand der Erfindung sind bei Temperaturen von oberhalb 5°C flüssige Polyisocyanatmischungen aus 4,4'-Diphenylmethandiisocyanat, gekennzeichnet durch
A) einen NCO-Gehalt von 14,5 bis 24 Gew.-%
und
B) einen ausschließlichen Gehalt an Allophanatgruppen (berechnet als C₂HN₂O₃, Molekulargewicht = 101) von 7,7 bis 14,5 Gew.-%.
Gegenstand der Erfindung ist auch ein Verfahren zur Herstellung dieser erfindungsgemaße Polyisocyanatmischungen, welches dadurch gekennzeichnet ist, daß man 4,4'-Diphenylmethandiisocyanat unter intermediärer Urethanbildung mit einwertigen Alkoholen mit 4 bis 16 Kohlenstoffatomen pro Molekül unter Einhaltung eines NCO/OH-Äquivalentverhältnisses von 5:1 bis 8,5:1 bei bis zu 160°C liegenden Temperaturen zur Reaktion bringt, wobei man die Umsetzung spätestens nach erfolgter Urethanbildung in Gegenwart eines die Allophanatbildung fördernden Katalysators durchführt.

Gegenstand der Erfindung ist schließlich auch die Verwendung der erfindungsgemäßen Polyisocyanatmischungen als Polyisocyanat-Komponente in Zweikomponenten-Polyurethan-Beschichtungsmitteln.

Allophanatgruppen aufweisende Polyisocyanatgemische sind bereits prinzipiell bekannt und beispielsweise in US-PS 3 769 318 oder GB-PS 994 890 beschrieben. Obwohl auch 4,4'-Diphenylmethandiisocyanat als geeignetes Ausgangsmaterial beiläufig erwähnt wird, kann gesagt werden, daß diesen Veröffentlichungen kein Hinweis darauf zu entnehmen ist, ob und unter welchen Bedingungen flüssige, kristallisationsstabile Polyisocyanatmischungen durch Modifizierung von 4,4'-Diphenylmethandiisocyanat hergestellt werden können.

Ausgangsmaterialien für das erfindungsgemäße Verfahren sind neben 4,4'-Diphenylmethandiisocyanat einwertige, vorzugsweise Ethergruppen-freie Alkohole mit 4 bis 16, vorzugsweise 4 bis 10 Kohlenstoffatomen pro Molekül. Bevorzugt werden lineare, d.h. unverzweigte, bei Raumtemperatur flüssige, Ethergruppen-freie einwertige aliphatische Alkohole mit 4 bis 10 Kohlenstoffatomen eingesetzt. Mischungen verschiedener derartiger Alkohole können selbstverständlich ebenfalls verwendet werden.

Geeignete Alkohole sind beispielsweise n-Butanol, i-Butanol, n-Pentanol, n-Hexanol, 2-Ethylhexanol, n-Octanol, n-Decanol, n-Dodecanol oder n-Hexadecanol. Prinzipiell ebenfalls verwendbar sind Ethergruppen aufweisende einwertige Alkohole, wie sie durch Ethoxylierung oder Propoxylierung der beispielhaft genannten einfachen Alkanole erhalten werden wie z.B. Diethylenglykolmonobutylether. Die Verwendung derartiger Ethergruppen aufweisender Monoalkohole ist jedoch wegen der erfindungsgemäß gerade angestrebten Abwesenheit von Ethersauerstoffatomen in den Verfahrensprodukten keineswegs bevorzugt.

Die Umsetzung erfolgt zumindest im Anschluß an die intermediäre Urethanbildung in Gegenwart von die Allophanatisierung fördernden Katalysatoren. Geeignete Katalysatoren sind beispielsweise in dem Reaktionsgemisch lösliche Metallverbindungen der 3. und 4. Hauptgruppe, sowie der 1., 2., 6., 7. und 8. Nebengruppe der in US-PS 3 769 318 genannten Art. Besonders bevorzugt werden Zinn(II)octoat oder Zinkacetylacetonat in Mengen von 20 bis 2000 ppm (Gewicht), vorzugsweise 20 bis 200 ppm (Gewicht), bezogen auf das Reaktionsgemisch eingesetzt. Die Anwesenheit von alkylierend wirkenden Verbindungen während der Reaktion ist im Unterschied zur Empfehlung der US-PS 3 769 318 nicht erforderlich. Überraschenderweise enthalten die erfindungsgemäßen Polyisocyanatmischungen ausschließlich Allophanatstrukturen und keine Urethan-, Isocyanurat- und/oder Carbodiimidstrukturen. Dies wird durch 13-C-NMR-spektroskopische Untersuchungen zweifelsfrei belegt.

Das erfindungsgemäße Verfahren wird bei Temperaturen von bis zu 160°C durchgeführt. Die bevorzugte Temperatur bezüglich der Allophanatisierungsreaktion liegt bei 80 bis 120°C. Gemäß einer bevorzugten Arbeitsweise werden die erfindungsgemäßen Polyisocyanatgemische in einer 2-stufigen Verfahrensweise hergestellt. In einem ersten Verfahrensschritt wird geschmolzenes 4,4'-Diphenylmethandiisocyanat vorgelegt und bei einer Temperatur zwischen 40°C und 80°C, vorzugsweise 50°C und 70°C der Monoalkohol zugetropft. Nach Erreichen des für die Urethanbildung berechneten NCO-Gehaltes wird im zweiten Verfahrensschritt der Katalysator zugesetzt und anschließend die Temperatur auf 80 bis 160°C, vorzugsweise 80 bis 120°C erhöht, wobei die Reaktion bei Erreichen des für die Allophanatbildung berechneten NCO-Gehaltes vorzugsweise durch Zugabe eines Katalysatorengifts abgebrochen wird. Geeignete Katalysatorengifte sind beispielsweise alkylierend oder acylierend wirkende Verbindungen wie p-Toluolsulfonsäuremethylester, Dimethylsulfat, Benzoylchlorid oder Isophthalsäuredichlorid, die vorzugsweise in mindestens äquimolaren Mengen, bezogen auf die Menge des eingesetzten Katalysators, eingesetzt werden.

Die erfindungsgemäßen Polyisocyanatgemische sind fast farblose und flüssige Gemische mit überraschend niedrigen Viskositäten zwischen 200 und 5000 mPa.s (23°C), wobei die Viskosität mit steigender Menge an Allophanatstrukturen zunimmt. Der NCO-Gehalt der erfindungsgemäßen Produktgemische liegt bei 14,5 bis 24 Gew.-%.

Die erfindungsgemäßen Polyisocyanatgemische zeigen eine hervorragende Kristallisationsbeständigkeit bei tiefen Temperaturen, Produktgemische mit einem hohen Anteil an Allophanatstrukturen zeigen auch bei Temperaturen unter 0°C keinerlei Kristallisation.

Die erfindungsgemäßen Polyisocyanatmischungen sind geeignete Ausgangsverbindungen zur Herstellung von Polyurethankunststoffen im allgemeinen, bevorzugt werden die Gemische jedoch zur Herstellung lösemittelfreier Polyurethan-Beschichtungssysteme eingesetzt. Zur Herstellung solcher Beschichtungssysteme werden die erfindungsgemäßen Polyisocyanatgemische mit aus der Polyurethanchemie an sich bekannten lösemittelfreien Polyhydroxylverbindungen bzw. Gemischen derselben kombiniert, wobei vorzugsweise die Mengenverhältnisse der Einzelkomponenten einem NCO/OH-Äquivalentverhältnis von 0,8:1 bis 1,5:1 entsprechen. Geeignete Polyhydroxylverbindungen sind beispielsweise Polyether-, Polyester- und/oder Polycarbonatpolyole.

Wegen der blasenfreien Aushärtung in dicker Schicht werden die erfindungsgemäßen Polyisocyanatmischungen bevorzugt mit Ricinusöl bzw. Abmischungen von Ricinusöl mit Keton-Formaldehydkondensaten und/oder Polyesterpolyolen und/oder Polyetherpolyolen kombiniert.

Aus derartigen Kombinationen erhält man transparente, farbhelle Beschichtungen von hoher Härte und guter Abriebbeständigkeit. Die Härte der erhaltenen Beschichtungen ist dabei höher als die Härte von analogen mit 4,4'-Diphenylmethandiisocyanat gehärteten Beschichtungen. Der für die meisten Anwendungen erforderliche Zusatz von Pigmenten und anderen Hilfs- und Zusatzmitteln wie Füllstoffen, Verlaufshilfsmitteln usw. wird vorzugsweise so vorgenommen, daß diese Zusatzmittel in eine der Ausgangskomponenten eingemischt werden. Die die erfindungsgemäßen Polyisocyanatmischungen enthaltenden Beschichtungsmaterialien können nach an sich bekannten Methoden wie beispielsweise durch Spritzen, Streichen, Tauchen, Fluten oder mit Hilfe von Walzen oder Rakeln auf beliebige Substrate ein- oder mehrschichtig aufgetragen werden. Als Substrate eignen sich beispielsweise Metall, Holz, Glas, Stein, keramische Materialien, Beton, härte und flexible Kunststoffe, Textilien, Leder oder Papier. Die Substrate können selbstverständlich vor der Applikation mit den üblichen Grundierungen versehen sein.

Die nachfolgenden Beispiele dienen zur weiteren Erläuterung der Erfindung. Alle Prozentangaben beziehen sich auf das Gewicht. Das in den nachfolgenden Beispielen eingesetzte 4,4'-Diphenylmethandiisocyanat stellt ein technisches Produkt mit einem Gehalt von 99,0 Gew.-% des 4,4'-Isomeren dar.

### Beispiel 1

### Herstellung einer Polyisocyanatmischung

1000 g geschmolzenes 4,4'-Diphenylmethandiisocyanat werden vorgelegt. Bei einer Temperatur von 60°C tropft man 74 g n-Butanol (NCO/OH-Verhältnis 8:1) langsam zu und rührt bei dieser Temperatur bis der für die Urethanbildung berechnete NCO-Gehalt von 27,4 % erreicht ist. Danach versetzt man mit 0,1 g Zinkacetylacetonat und erhöht die Temperatur auf 100°C. Man rührt bei dieser Temperatur bis der für die Allophanatbildung berechnete NCO-Gehalt von 23,5 % erreicht ist. Danach beendet man die Reaktion durch Zugabe von 0,1 g Benzoylchlorid. Man erhält so ein flüssiges, blaßgelb gefärbtes Polyisocyanatgemisch mit einer Viskosität von 300 mPa.s (23°C). Das Produkt ist bei einer Temperatur von 5°C über eine Lagerdauer von mehreren Wochen kristallisationsstabil, bei einer Lagerung bei 0°C trübt das Produkt nach ca. 5 Tagen ein. Das Produkt enthält einen berechneten Anteil an Allophanatgruppen von 9,4 %. Das 13C-NMR-Spektrum zeigt im Carbonylbereich lediglich Signale bei 156 ppm und 151,5 ppm entsprechend Allophanatstrukturen sowie 120 ppm entsprechend Isocyanatstrukturen.

### Beispiel 2

### Herstellung einer Polyisocyanatmischung

Entsprechend der Vorschrift aus Beispiel 1 werden 875 g 4,4'-Diphenylmethandiisocyanat mit 74 g n-Butanol umgesetzt (NCO/OH-Verhältnis 7:1). Das erhaltene Polyisocyanatgemisch hat eine Viskosität von 1100 mPa.s (23°C) und einen NCO-Gehalt von 22,0 %. Das Produkt ist bei eienr Temperatur von 0°C über eine Lagerdauer von mehreren Wochen kristallisationsstabil. Das Produkt enthält einen berechneten Anteil an Allophanatgruppen von 10,6 %.

### Beispiel 3

### Herstellung einer Polyisocyanatmischung

Entsprechend der Vorschrift aus Beispiel 1 werden 750 g 4,4'-Diphenylmethandiisocyanat mit 74 g n-Butanol umgesetzt (NCO/OH-Verhältnis 6:1). Das erhaltene Polyisocyanatgemisch hat eine Viskosität von 5000 mPa.s (23°C) und einen NCO-Gehalt von 20,2 %. Das Produkt ist bei einer Temperatur von 0°C über eine Lagerdauer von mehreren Wochen kristallisationsstabil. Das Produkt enthält einen berechneten Anteil an Allophanatgruppen von 12,2 %.

### Beispiel 4

### Herstellung einer Polyisocyanatmischung

Entsprechend der Vorschrift aus Beispiel 1 werden 812 g 4,4'-Diphenylmethandiisocyanat mit 66,6 g n-Butanol im Gemisch mit 15,8 g 1-Decanol umgesetzt (NCO/OH-Verhältnis 6,5:1). Das erhaltene Polyisocyanatgemisch hat eine Viskosität von 1800 mPa.s (23°C) und einen NCO-Gehalt von 21,1 %. Das Produkt ist bei einer Temperatur von 0°C über eine Lagerdauer von mehreren Wochen kristallisationsstabil. Das Produkt enthält einen berechneten Anteil an Allophanatgruppen von 11,3 %.

### Beispiel 5

### Herstellung einer Polyisocyanatmischung

Entsprechend der Vorschrift aus Beispiel 1 wserden 875 g 4,4'-Diphenylmethandiisocyanat mit 102 g n-Hexanol umgesetzt (NCO/OH-Verhältnis 7:1). Das erhaltene Polyisocyanatgemisch hat eine Viskosität von 680 mPa.s (23°C) und einen NCO-Gehalt von 21,4 %. Das Produkt ist bei einer Temperatur von 0°C über eine Lagerdauer von mehreren Wochen kristallisationsstabil. Das Produkt enthält einen berechneten Anteil an Allophanatstrukturen von 10,3 %.

### Beispiel 6

### Herstellung einer lösemittelfreien Zweikomponenten-Polyurethanbeschichtung

180 g Polyisocyanatmischung aus Beispiel 1 werden mit 350 g eines handelsüblichen Polyols auf Ricinusölbasis (®Desmophen 1150, Handelsprodukt der Bayer AG) innig vermischt. Die Verarbeitungszeit der Mischung beträgt 45 Minuten. Ein auf eine Glasplatte aufgegossener Film trocknet zu einer blasenfreien, zähharten, hochglänzenden und abrasionsfesten Beschichtung. An einem gegossenen Prüfkörper wird nach 7-tägiger Lagerung bei Raumtemperatur eine Shore D-Härte von 70 gemessen.

## Patentansprüche

1. Bei Temperaturen von oberhalb 5°C flüssige Polyisocyanatmischungen aus 4,4'-Diphenylmethandiisocyanat oder dessen Gemischen mit bis zu 1,9 Gew.% an 2,4'- und/oder bis zu 0.5 Gew.% an 2,2'-Diphenylmethandiisocyanat, wobei sich diese Prozentangabern jeweils auf das Gesamtgemisch beziehen, gekennzeichnet durch
A) einen NCO-Gehalt von 14,5 bis 24 Gew.-%
und
B) ausschließlich einen Gehalt an Allophanatgruppen (berechnet als C₂HN₂O₃, Molekulargewicht = 101) von 7,7 bis 14,5 Gew.-%.

2. Polyisocyanatmischungen gemäß Anspruch 1, dadurch gekennzeichnet, daß sie unter intermediärer Urethanbildung hergestellte Umsetzungsprodukte von
a) 4,4'-Diphenylmethandiisocyanat
mit
b) einwertigen Alkoholen mit 4 bis 16 Kohlenstoffatomen pro Molekül
unter Einhaltung eines NCO/OH-Äquivalentverhältnisses von 5:1 bis 8,5:1 darstellen.

3. Polyisocyanatmischungen gemäß Anspruch 2, dadurch gekennzeichnet, daß sie Umsetzungsprodukte von
a) 4,4'-Diphenylmethandiisocyanat
mit
b) linearen, einwertigen Alkoholen mit 4 bis 10 Kohlenstoffatomen
darstellen.

4. Verfahren zur Herstellung von bei Temperaturen oberhalb 5°C flüssigen Polyisocyanatmischungen aus 4,4'-Diphenylmethandiisocyanat gemäß Anspruch 1, dadurch gekennzeichnet, daß man 4,4'-Diphenylmethandiisocyanat unter intermediärer Urethanbildung mit einwertigen Alkoholen mit 4 bis 16 Kohlenstoffatomen pro Molekül unter Einhaltung eines NCO/OH-Äquivalentverhältnisses von 5:1 bis 8,5:1 bei bis zu 160°C liegenden Temperaturen zur Reaktion bringt, wobei man die Umsetzung spätestens nach erfolgter Urethanbildung in Gegenwart eines die Allophanatbildung fördernden Katalysators durchführt.

5. Verfahren gemäß Anspruch 4, dadurch gekennzeichnet, daß man als die Allophanatbildung fördernde Katalysatoren in dem Reaktionsgemisch lösliche Verbindungen der 3. oder 4. Hauptgruppe oder der 1., 2., 6., 7. oder 8. Nebengruppe des Periodensystems der Elemente verwendet.

6. Verfahren gemäß Anspruch 4 und 5, dadurch gekennzeichnet, daß man als Katalysator Zinn(II)octoat oder Zinkacetylacetonat verwendet.

7. Verwendung der Polyisocyanatmischungen gemäß Anspruch 1 bis 3 als Polyisocyanatkomponente in Zweikomponenten-Polyurethan-Beschichtungsmitteln.

## Claims

1. Polyisocyanate mixtures consisting of 4,4'-diphenylmethane diisocyanate, or mixtures thereof with up to 1.9 wt.% of 2,4'- and/or up to 0.5 wt.% of 2,2'-diphenylmethane diisocyanate, each of these percentages being based on the mixture overall, which are liquid at temperatures of over 5°C, characterised by
A) an NCO content of 14.5 to 24 wt.%
and
B) an exclusive content of allophanate groups (calculated as C₂HN₂O₃, molecular weight = 101) of 7.7 to 14.5 wt.%.

2. Polyisocyanate mixtures according to claim 1, characterised in that they represent reaction products of
a) 4,4'-diphenylmethane diisocyanate
with
b) monohydric alcohols with 4 to 16 carbon atoms per molecule
produced with intermediate urethane formation, maintaining an NCO/OH equivalent ratio of 5:1 to 8.5:1.

3. Polyisocyanate mixtures according to claim 2, characterised in that they represent reaction products of
a) 4,4,-diphenylmethane diisocyanate
with
b) linear, monohydric alcohols with 4 to 10 carbon atoms.

4. Process for the production of polyisocyanate mixtures consisting of 4,4'-diphenylmethane diisocyanate according to claim 1 which are liquid at temperatures of over 5°C, characterised in that 4,4'-diphenylmethane diisocyanate is reacted at temperatures of up to 160°C with monohydric alcohols with 4 to 16 carbon atoms per molecule maintaining an NCO/OH equivalent ratio of 5:1 to 8.5:1 with intermediate urethane formation, the conversion being carried out at the latest after urethane formation has occurred, in the presence of a catalyst which promotes allophanate formation.

5. Process according to claim 4, characterised in that compounds of main groups 3 or 4 or of subgroups 1, 2, 6, 7 or 8 of the periodic system of elements, which are soluble in the reaction mixture, are used as the allophanate formation-promoting catalysts.

6. Process according to claims 4 and 5, characterised in that tin (II) octoate or zinc acetylacetonate is used as catalyst.

7. Use of the polyisocyanate mixtures according to claims 1 to 3 as the polyisocyanate component in two-component polyurethane coating compounds.

## Revendications

1. Mélanges de polyisocyanates liquides au-dessus de 5°C de 4,4'-diphénylméthanediisocyanate, ou ses mélanges avec jusqu'à 1,9% en poids de 2,4'- et/ou jusqu'à 0,5% en poids de 2,2'-diphénylméthanediisocyanate, où ces données en pourcentage se rapportent chaque fois au mélange total, caractérisés par :
a) une teneur en NCO de 14,5 à 24% en poids,
et
b) une teneur exclusive en radicaux allophanate (calculés comme C₂HN₂O₃, poids moléculaire = 101) de 7,7 à 14,5% en poids.

2. Mélanges de polyisocyanates suivant la revendication 1, caractérisés en ce qu'ils représentent les produits de réaction préparés avec formation intermédiaire d'uréthanne,
a) du 4,4'-diphénylméthanediisocyanate, avec
b) des alcools monovalents ayant 4 à 16 atomes de carbone par molécule
en respectant un rapport d'équivalents NCO/OH compris dans l'intervalle allant de 5:1 à 8,5:1.

3. Mélanges de polyisocyanates suivant la revendication 2, caractérisés en ce qu'ils représentent les produits de réaction
a) du 4,4'-diphénylméthanediisocyanate, avec
b) des alcools monovalents, linéaires ayant 4 à 10 atomes de carbone.

4. Procédé de préparation de mélanges de polyisocyanates liquides au-dessus de 5°C de 4,4'-diphénylméthanediisocyanate suivant la revendication 1, caractérisé en ce que l'on fait réagir le 4,4'-diphénylméthanediisocyanate avec formation intermédiaire d'uréthanne, avec des alcools monovalents ayant 4 à 16 atomes de carbone par molécule, en respectant un rapport d'équivalents NCO/OH compris dans l'intervalle allant de 5:1 à 8,5:1, à des températures allant jusqu'à 160°C, où l'on réalise la transformation au plus tard après la formation réussie d'uréthanne, en présence d'un catalyseur accélérant la formation d'allophanate.

5. Procédé suivant la revendication 4, caractérisé en ce que l'on utilise comme catalyseur accélérant la formation d'allophanate, des composés solubles dans le mélange réactionnel, de métaux du groupe principal 3 ou 4 ou du groupe secondaire 1, 2, 6, 7 ou 8 du tableau périodique des éléments.

6. Procédé suivant les revendications 4 et 5, caractérisé en ce que l'on utilise comme catalyseur, l'octoate d'étain (II) ou l'acétylacétonate de zinc.

7. Utilisation des mélanges de polyisocyanates suivant les revendications 1 à 3, comme composant polyisocyanate dans des agents de revêtement à deux composants, de type polyuréthanne.
